## Europäisches Patentamt
## European Patent Office
## Office européen des brevets

(11) Veröffentlichungsnummer: **0 044 968**
**B1**

(12) **EUROPÄISCHE PATENTSCHRIFT**

(45) Veröffentlichungstag der Patentschrift:
**15.02.84**

(21) Anmeldenummer: **81105197.8**

(22) Anmeldetag: **04.07.81**

(51) Int. Cl.³: **C 07 D 295/02, C 07 D 211/14, C 07 D 211/20, C 07 D 207/06, C 07 D 207/08, C 07 D 211/02**

(54) **Verfahren zur Herstellung von N-Alkylpiperidinen und N-Alkylpyrrolidinen.**

(30) Priorität: **26.07.80 DE 3028384**

(43) Veröffentlichungstag der Anmeldung:
**03.02.82 Patentblatt 82/5**

(45) Bekanntmachung des Hinweises auf die Patenterteilung:
**15.02.84 Patentblatt 84/7**

(84) Benannte Vertragsstaaten:
**BE CH DE FR GB IT LI NL**

(56) Entgegenhaltungen:
**DE - A - 2 617 402**
**DE - A - 2 813 162**
**DE - B - 1 670 056**
**DE - B - 2 514 004**
**FR - A - 1 475 961**
**GB - A - 971 187**

(73) Patentinhaber: **BASF Aktiengesellschaft, Carl-Bosch-Strasse 38, D-6700 Ludwigshafen (DE)**

(72) Erfinder: **Rebafka, Walter, Dr., Schuetzenstrasse 4, D-6901 Eppelhelm (DE)**
Erfinder: **Schossig, Juergen, Dr., Rheinparkstrasse 3, D-6800 Mannheim (DE)**
Erfinder: **Reiss, Wolfgang, Dr., Bannwasserstrasse 70, D-6700 Ludwigshafen (DE)**
Erfinder: **Voges, Dieter, Dr., Richard-Wagner-Strasse 28, D-6800 Mannheim (DE)**

## Verfahren zur Herstellung von N-Alkylpiperidinen und N-Alkylpyrrolidinen

Während die Hydrierung am Stickstoffatom unsubstituierter cyclischer Imide von Mono- und Dicarbonsäuren weitgehend bekannt ist und wohl z.T. auch in technischem Massstab stattfindet (vgl. z.B. FR-PS 1 475 961; ähnlich auch DE-OS 25 14 004 – Hydrierung eines Dicarbonsäuredinitrils), ist die Herstellung N-substituierter Pyrrolidine und Piperidine bisher nur entweder durch hydrierende N-Alkylierung unsubstituierter Succin- bzw. Glutarimide in Alkoholen (die somit Lösungs- und Alkylierungsmittel sind) oder durch Hydrierung der N-Alkyl-dicarbonsäureimide mit speziellen Katalysatoren in Dioxan als Lösungsmittel gelungen (vgl. SU-PS 259 889 bzw. Paden und Adkins, JACS 58, 2487 (1936)).

Ein Sonderfall, der nicht verallgemeinert werden kann, ist die Hydrierung von Butyrolactam (Pyrrolidon) an einem Kobalt-Sintermetallkatalysator, die zum N-Butylpyrrolidin führt (vgl. DE-OS 16 70 056), somit von einer komplizierten Umlagerungsreaktion unbekannten Ablaufs begleitet ist.

Es besteht daher Bedarf an einem Verfahren zur Herstellung von N-Alkylpyrrolidinen und N-Alkylpiperidinen durch Hydrierung der entsprechenden Dicarbonsäureimide, das ohne fremde Lösungsmittel oder ähnliche Hilfsmittel (ausser dem Katalysator) auskommt und bei den hohen Umsatzgeschwindigkeiten und Ausbeuten erzielt werden.

Bei der näheren Untersuchung des Reaktionsverlaufs hat es sich nun gezeigt, dass bei unvollständigem Umsatz in Gegenwart eines geeigneten Katalysators Zwischenprodukte mit sehr hohem Siedepunkt entstehen, die jedoch nach einer entsprechenden Trennoperation zurückgeführt und vollständig umgesetzt werden können. Bei ihrer Abtrennung wird ausser den Zielprodukten u.a. Wasser entfernt.

Erfindungsgegenstand ist daher ein Verfahren zur Herstellung von N-Alkylpiperidinen und N-Alkylpyrrolidinen durch Hydrierung der entsprechenden Dicarbonsäureimide in Gegenwart eines Hydrierkatalysators, bei dem man die Hydrierung fortlaufend an einem fest angeordneten Kobaltkatalysator vornimmt, wobei man den Umsatz je Durchgang auf 10 bis 90% einstellt und das den Katalysator verlassende Reaktionsgemisch in teilumgesetzte Produkte, Wasser und N-Alkylpiperidin bzw. N-Alkylpyrrolidin trennt und die teilumgesetzten Produkte dem Katalysator wieder zuführt. Man führt zweckmässig das Reaktionsgemisch mit geeigneter Geschwindigkeit über den fest angeordneten Katalysator im Kreis und trennt vollständig und unvollständig bzw. nicht umgesetzte Gemischbestandteile voneinander und von enthaltenem Wasser. Für die Trennung eignen sich z.B. Dünnfilmverdampfer.

Der Umsatz je Durchgang wird zweckmässig auf 50% oder weniger eingestellt.

Die verwendeten Kobalt-Katalysatoren scheinen im Gegensatz zu den von Adkins angegebenen Kupferchromit-Katalysatoren auch in Gegenwart von Wasser wirksam zu sein und machen somit die Verwendung wasserentziehender Lösungsmittel (vgl. Adkins, JACS a.a.O.) entbehrlich.

Bevorzugt ist die Verwendung von sog. Vollkontakten, d.h. trägerfreien Katalysatoren, die neben Kobalt vorzugsweise geringe Mengen von z.B. Kupfer, Mangan und Phosphorsäure enthalten. Das Verfahren ist aber auch mit Katalysatoren ausführbar, die Kobalt und ggf. weitere Bestandteile auf einem Träger enthalten. Der Träger kann z.B. Aluminiumoxid sein.

Die Katalysatoren werden in katalytisch wirksamen Mengen gebraucht, die von der jeweils angewandten Technologie in bekannter Weise abhängen.

Die Reaktion läuft bei etwa 150 bis 250 °C und einem Druck von z.B. 50 bis 500 bar mit befriedigender Geschwindigkeit ab. Die Ausbeute erreicht im allgemeinen mehr als 70% der rechnerisch möglichen.

N-Alkylpyrrolidine bzw. -piperidine mit z.B. 1 bis 20 C-Atomen in der Alkylgruppe werden als Vorprodukte für Pflanzenschutzmittel, als Katalysatoren für die Herstellung von Polyurethan-Kunststoffen und z.B. als spezielle Basen bei Arzneimitteln verwendet. Sie können auch an den C-Atomen als Ringgerüst weiter substituiert sein, z.B. durch Alkyl- oder Alkoxy-methylgruppen.

Die in den angeführten Versuchen gezeigten Betriebsweisen geben z.T. nicht das vollständige Verfahren wieder, sondern arbeiten z.B. in einzelnen Fällen ohne Rückführung, um die Zusammensetzung des Reaktionsgemisches bei einfachen, d.h. einmaligem Durchgang zu ermitteln. Beispiel 1 und 2 geben vollständige Betriebszyklen wieder.

Versuche 1 bis 3

Die Versuche wurden in einem üblichen Hochdruck-Versuchsreaktor in Rohrform bei 250 bar Wasserstoffdruck vorgenommen, der jeweils mit einem aus 64% Kobaltoxid, 18% Kupfer(II)oxid, 7% Mangan(II, III)oxid und 4% Phosphorsäure hergestellten Vollkatalysator in Strangform (6 mm Durchmesser) gefüllt war.

Verschiedene Betriebsweisen (aufwärts, abwärts), Mengen usw. wurden angewandt. Die Bedingungen und Ergebnisse sind in der Tabelle angegeben.

Tabelle

| Versuch Nr. (Betriebs- richtung) | Reaktor- grösse (1) | Art [+] und Menge des Ausgangsmaterials | | Temp. (°C) | Umsatz je Durchgang (%) | Ausbeute (Gesamt- umsatz) (%) |
|---|---|---|---|---|---|---|
| 1 (↓) | 8 | 1.5 l/h | 33% N MS 67% N MG | 230 | | 75 (100) 70 ( 80) |
| 2 (↑) | 2.2 | wie 1 | | 240 | | 72 (100) 75 (100) |
| 3 (↓) | 2.2 | 0.1 l/h | 20% N ES 30% N EG 50% N EPP [++] | 200 | | 60 (100) 80 (100) |

[+] NMS = N-Methylsuccinimid; NMG = N-Methyl-glutarimid; NEG = N-Äthylglutarimid; NES = N-Äthylsuccinimid; NEPP = N-Äthylpiperidin
[++] als Lösungsmittel

Versuch 4

In der für die Versuche 2 und 3 verwendeten Vorrichtung wurden 2.2 l eines Kobalt-Katalysa- tors angeordnet, der 17% Kobaltoxid auf einem Aluminiumoxid-Träger in Form 4 mm starker Stränge enthielt.

Nach üblicher Vorbehandlung mit Wasserstoff wurde der Katalysator bei 210 °C mit stündlich 100 ml eines Gemisches von N-Methylsuccinimid (NMS) und N-Methylglutarimid (NMG) im Gewichtsverhältnis 35:65 beschickt, das etwa 6% Wasser enthielt. Der Wasserstoffdruck wurde auf 250 bar gehalten.

Unter diesen Bedingungen wurde ein Umsatz von jeweils 76% beobachtet; die Ausbeute bezogen auf umgesetzte Ausgangsstoffe, erreichte 70%.

Versuch 5

Es wurde verfahren wie vorstehend beschrieben unter Verwendung eines aus 6 mm starken Strängen von reinem Kobaltoxid bestehenden Katalysators.

Das zugeführte Gemisch (NMS, NMG im Verhältnis 4:6) enthielt 7% Wasser.

Bei einem Umsatz von jeweils 50% wurde eine Selektivität von 68 (NMS) bzw. 70% (NMG) erreicht.

Beispiel 1

Ein senkrecht stehender, rohrförmiger Hochdruckreaktor für fortlaufenden Betrieb wurde mit 10 l des in den Beispielen 1 bis 3 beschriebenen Katalysators gefüllt und eine Reaktionstemperatur von 210 °C bei einem Wasserstoffdruck von 250 bar eingestellt. Man führte von oben stündlich 3 l des Rohgemisches von Beispiel 5 (NMS, NMG; 4:6) zu und mischte diesem zunächst von der ablaufenden Flüssigkeit soviel bei, dass eine Flächenbelastung von 30 (m³/m².h, bezogen auf den leeren Reaktor) erzielt wurde. Die im Kreis geführte Flüssigkeit wurde soweit gekühlt, dass die Reaktionstemperatur gehalten werden konnte.

Die restliche ablaufende Flüssigkeitsmenge wurde in einem bei atmosphärischem Druck betriebenen Fallfilmverdampfer auf 180 °C erhitzt, wobei Wasser und leichtsiedende Produkte ent- fernt wurden. Ihre Menge beträgt rund 28% des Ablaufs.

Die schwersiedenden Bestandteile erwiesen sich als praktisch undestillierbar (bei 0.1 mbar höher als 200 °C); sie wurden mit frischem NMS/ NMG-Gemisch versetzt und dem Reaktor wieder zugeführt. Im zeitlichen Gleichgewicht erwies sich die Menge dieser schwersiedenden Produkte als konstant, woraus sich ergibt, dass sie als Zwischenprodukte anzusehen sind, die bei weiterer Umsetzung ebenfalls in Zielprodukte übergehen.

Das aus dem Fallfilmverdampfer erhaltene Destillat enthielt 17 Gew.-Teile – neben Wasser und Nebenprodukten – 4 Gew.-Teile N-Methylpyrrolidin und 6.4 Gew.-Teile N-Methylpiperidin. Extrapoliert auf vollständigen Umsatz errechnet sich hieraus die Selektivität (Ausbeute) zu jeweils rund 85%.

Beispiel 2

Ein senkrecht stehender, rohrförmiger Hochdruckreaktor für kontinuierlichen Betrieb wurde mit 3 l des in Beispiel 1 bis 3 beschriebenen Katalysators gefüllt und bei 210 °C sowie einem Wasserstoffdruck von 250 bar in der folgenden Weise betrieben. Man führt von oben stündlich 1,2 l ei- nes Gemisches dem Reaktor zu, welches aus 0,4 l N-Methylsuccinimid und 0,8 l des Destillations- rückstandes bestand, der bei der im nachfolgen- den beschriebenen destillativen Aufarbeitung an- fiel. Zur Abführung der anfallenden Reaktionswärme wurde von der ablaufenden Flüssigkeit soviel auf den Kopf des Reaktors zurückgeführt, dass eine Flächenbelastung von 30 m³/m²·h (bezogen auf leeren Reaktorquerschnitt) erzielt wird.

Die restliche ablaufende Flüssigkeit wird in einem bei Normaldruck betriebenen Fallfilmverdampfer auf 170 °C erhitzt, wobei im wesentlichen das gebildete Wasser sowie als Wertprodukt das N-Methylpyrrolidin abdestillieren. Unter diesen Bedingungen fielen im kontinuierlichen Betrieb stündlich 0,5 l Destillat sowie 0,8 l eines schwersiedenden Destillationsrückstandes an, welcher im oben angegebenen Verhältnis mit frischem N-Methylsuccinimid vermischt und dem Reaktor wieder zugeführt wurde.

Im zeitlichen Gleichgewicht erweist sich die Menge dieser schwersiedenden Produkte als konstant, woraus sich ergibt, dass sie als Produkte anzusehen sind, die bei weiterer Umsetzung ebenfalls zum Zielprodukt umgesetzt werden. Beachtenswert ist, dass dieser Destillationsrückstand nur weniger als 5% des Ausgangsmaterials enthält und auch im Destillat dieses nicht auftritt.

Die Analyse des anfallenden Destillates ergab eine Ausbeute von 78% an N-Methylpyrrolidin bezogen auf die stündlich zugeführte Menge von 0,4 l N-Methylsuccinimid.

## Patentansprüche

1. Verfahren zur Herstellung von N-Alkylpiperidinen und N-Alkylpyrrolidinen durch Hydrierung der entsprechenden Dicarbonsäureimide in Gegenwart eines Hydrierkatalysators, dadurch gekennzeichnet, dass man die Hydrierung fortlaufend an einem fest angeordneten Kobaltkatalysator vornimmt, wobei man den Umsatz je Durchgang auf 10 bis 90% einstellt und das den Katalysator verlassende Reaktionsgemisch in teilumgesetzte Produkte, Wasser und N-Alkylpiperidin bzw. N-Alkylpyrrolidin trennt und die teilumgesetzten Produkte dem Katalysator wieder zuführt.

2. Verfahren nach Anspruch 1, dadurch gekennzeichnet, dass man einen Kobaltkatalysator verwendet, der ausserdem wirksame Mengen Kupfer, Mangan und Phosphorsäure enthält und im wesentlichen trägerfrei ist.

3. Verfahren nach Anspruch 1 oder 2, dadurch gekennzeichnet, dass man das Gemisch aus Ausgangsstoff und Reaktionsprodukt als wesentliches Lösungsmittel verwendet, bzw. kein zusätzliches Lösungsmittel verwendet.

## Revendications

1. Procédé pour la préparation de N-alcoylpipéridines et de N-alcoylpyrrolidines par hydrogénation des imides d'acides dicarboxyliques correspondants en présence d'un catalyseur d'hydrogénation, caractérisé en ce qu'on procède à l'hydrogénation en continu sur un catalyseur au cobalt placé en situation fixe, en réglant le degré de transformation par passage à 10 à 90%, en séparant le mélange réactionnel qui quitte le catalyseur en produits partiellement transformés, en eau et en N-alcoylpipéridine ou N-alcoylpyrrolidine, et en renvoyant vers le catalyseur les produits partiellement transformés.

2. Procédé selon la revendication 1, caractérisé en ce qu'on utilise un catalyseur au cobalt qui contient en outre des quantités actives de cuivre, de manganèse et d'acide phosphorique et qui est essentiellement sans support.

3. Procédé selon la revendication 1 ou 2, caractérisé en ce qu'on utilise comme solvant principal le mélange de substance de départ et de produit de la réaction, c'est-à-dire qu'on n'utilise pas de solvant supplémentaire.

## Claims

1. A process for the preparation of N-alkylpiparidines and N-alkylpyrrolidines by hydrogenating the corresponding dicarboxylic acid imides in the presence of a hydrogenation catalyst, wherein the hydrogenation is carried out continuously over a fixed bed cobalt catalyst, the conversion being adjusted to 10 to 90% per pass, and the reaction mixture leaving the catalyst being separated into partially reacted products, water and the N-alkylpiperidine or N-alkylpyrrolidine, and the partially reacted products being recycled to the catalyst.

2. A process as claimed in claim 1, wherein a cobalt catalyst is used which additionally contains effective amounts of copper, manganese and phosphoric acid, and is essentially unsupported.

3. A process as claimed in claim 1 or 2, wherein the mixture of starting material and reaction product is used as sole solvent, i.e. no additional solvent is employed.